# EUROPEAN PATENT APPLICATION

(11) **EP 4 646 998 A1**
(43) Date of publication of application: **12.11.2025**
(21) Application number: 23914994.1
(22) Date of filing: 26.12.2023
(51) Int. Cl.: A61B 5/00, A61B 5/11, G16H 50/20

(54) **METHOD FOR EVALUATING AND ANALYZING MUSCULOSKELETAL INJURIES THROUGH 3D DYNAMIC JOINT RANGE OF MOTION TESTING AND POSTURAL ABNORMALITIES THROUGH STATIC POSTURE TESTING**

(30) Priority: 03.01.2023 KR 20230000891
(71) Applicant: Sym Healthcare Inc., Seoul 02796 (KR)
(72) Inventor: AHN, Junghoon, Namyangju-si, Gyeonggi-do 12113 (KR)
(74) Representative: González López-Menchero, Álvaro Luis
(86) International application number: PCT/KR2023/021535
(87) International publication number: WO 2024/147534

(57) **Abstract**

Proposed is a method for evaluating and analyzing musculoskeletal injuries through 3D dynamic joint range of motion testing, and postural abnormalities through static posture testing. In the method, compensatory movement (compensatory action) and Limitation Of Motion (LOM) are calculated by analyzing a position or changes in each joint along X, Y, and Z axes when motion injury testing or posture testing is performed, and a rehabilitation exercise and a preventive exercise suitable for each individual are recommended accordingly and guide videos are provided together, thereby increasing the participation rate and the efficiency of the rehabilitation exercise and the preventive exercise.

## Description

### Technical Field

The present disclosure relates to a method for evaluating and analyzing musculoskeletal injuries through 3D dynamic joint range of motion testing, and postural abnormalities through static posture testing. More particularly, the present disclosure relates to a method for evaluating and analyzing musculoskeletal injuries through 3D dynamic joint range of motion testing, and postural abnormalities through static posture testing, the method being configured for calculating compensatory movement (compensatory action) and limitation of motion by analyzing a position and changes in each joint along X, Y, and Z axes during motion injury testing or posture testing.

### Background Art

Due to changes in work patterns and lifestyle, such as performing repetitive tasks and operating smart devices for a long time, the occurrence of musculoskeletal diseases and the demand for healthcare services are increasing day by day.

As of 2019, 17.61 million people in Korea, which is one out of every three citizens, received treatment for musculoskeletal disorders, marking a 7.9% increase compared to 2009. Additionally, medical expenses related to musculoskeletal disorders have increased, accounting for 11% of total medical expenditures. Among musculoskeletal disorders, the rate of patients with cervical pain particularly related to the use of computers and smart devices has risen sharply over the past 10 years. This trend is well-documented in the press releases of the Health Insurance Review and Assessment Service.

Lack of awareness of correct movement and absence of related systems (solutions) are also contributing to this situation. In the past, the focus was primarily on quantitative measurement and evaluation of muscle dysfunction and body composition (body fat), with an emphasis on strength training and aerobic exercise. However, with the development of rehabilitation medicine and preventive medicine, the current approach has shifted towards both quantitative measurement and qualitative assessment of movement dysfunction, integrating the musculoskeletal and nervous systems, with a focus on exercise and rehabilitation. In the United States, experts are finding methods to analyze movement using the latest technologies and machines in order to alleviate pain and reduce the risk of injury. (Maury Hayashida et al. 2015)

One of the issues required to be solved is a problem of the current musculoskeletal examination and rehabilitation exercise prescription that are carried out subjectively and qualitatively through visual inspection and palpation on the basis of the expertise of individual musculoskeletal healthcare professionals. As healthcare services such as physical/rehabilitation therapy or exercise prescriptions are provided on the basis of qualitative assessments derived from the personal experience and expertise of professionals, the majority of people find it difficult to trust the data of the professionals due to subjective and qualitative evaluations, so that the collection of quantitative movement (posture and movement) data that guarantees measurement reliability is necessarily required.

By quantitatively measuring a three-dimensional movement of the musculoskeletal system according to a standardized protocol based on scientific evidence, the foundation for the development of new data-based services in the future is planned to be laid. Current musculoskeletal examination services primarily rely on two-dimensional posture assessments from the front or side views, so that the reliability and the scope of assessment items are limited. Therefore, the current musculoskeletal examination services only supplement existing visual inspections and palpation examinations. Accordingly, it is urgent to establish the foundation for developing new data-based services in the musculoskeletal healthcare field by quantitatively measuring three-dimensional musculoskeletal movements and accumulating musculoskeletal movement data.

By converting examinations and exercise coaching into data on the basis of quantitative data on musculoskeletal movements, the rapidly increasing demand for musculoskeletal healthcare services is aimed to be met by assisting in the decision-making of professionals in musculoskeletal healthcare services, thereby meeting the rapidly increasing demand for these services. The needs raised by service providers in the musculoskeletal healthcare field are to confirm the results of qualitative musculoskeletal examinations with quantitative data and to systematically provide healthcare services on the basis of the data. In order to definitely ensure the reliability of measured musculoskeletal posture and movement data so as to assist the direct examination process of medical professionals, it is necessary to help accurately understand the patient's condition through data generated by standardizing the examination process and method and to assist in decision-making about healthcare services.

A musculoskeletal examination and coaching system based on quantitative measurement technology for three-dimensional musculoskeletal movements is intended to be developed, and a musculoskeletal disease determination assistance system that presents disease groups predicted from disease-related movement characteristics is intended to be developed. By developing a system in which the movement of the musculoskeletal system in three dimensions based on a multi-view image taken simultaneously in several directions are measured while a subject performs a predetermined posture or a predetermined movement, movement characteristics highly related to disease determination in each posture or movement are analyzed and presented as data, expected disease groups estimated through clinical and scientific disease determination guidelines are also presented, so that the service provision process of musculoskeletal healthcare professionals is required to be assisted.

In the long term, it is intended to lay a foundation for developing data-based musculoskeletal health care services. By implementing a standardized musculoskeletal system examination so as to objectively understand the patient's musculoskeletal system state and by converting quantitative data on musculoskeletal movements and information on expected diseases predicted from movements into big data, a foundation for creating new service opportunities and expanding into various fields in the musculoskeletal healthcare field similar to the general health check-up field may be established.

Therefore, in order to solve the aforementioned problems, it has become necessary to conduct research on systems and methods that can estimate syndromes on the basis of quantitative data of musculoskeletal conditions while the recognition rate is increased.

### Disclosure

### Technical Problem

An objective of the present disclosure is intended to provide a method for evaluating and analyzing musculoskeletal injuries through 3D dynamic joint range of motion testing, and postural abnormalities through static posture testing, the method being configured for calculating compensatory movement (compensatory action) and Limitation Of Motion (LOM) by analyzing a position and changes in each joint along X, Y, and Z axes during motion injury testing or posture testing.

### Technical Solution

In order to achieve the objective of the present disclosure,
according to an aspect of the present disclosure, there is provided a method for evaluating and analyzing musculoskeletal injuries through 3D dynamic joint range of motion testing and postural abnormalities through static posture testing, the method including:
   (a) selecting, by a person through a testing type selection part provided by a server, at least one testing type for which the person intends to be tested;
   (b) photographing a movement and a motion of the person through a multiple viewpoints measurement device and transmitting photographed information to the server;
   (c) three-dimensionalizing (X, Y, and Z axes), by a person calculation part, information including information of a joint, a muscle, and a posture about the person on the basis of the testing type selected in the testing type selection part and on the basis of the information collected in the multiple viewpoints measurement device, and determining, by a data analysis part, whether the person has Limitation Of Motion (LOM) or compensatory movement, thereby determining whether there is a syndrome;
   (d) calculating, by a testing result calculation part, a testing result on the basis of information calculated in the data analysis part; and
   (e) recommending, through a smart exercise coaching part, a rehabilitation exercise or a preventive exercise on the basis of the testing result calculated in the data analysis part,
wherein the data analysis part includes: a motion injury detection part configured to detect a static posture of at least one of joints of the person and to detect whether there is an imbalance for each muscle that is estimated; and
a postural abnormality detection part configured to detect whether there is an imbalance for the static posture of at least one of the joints,
wherein the motion injury detection part includes:
   a LOM determination part configured to determine whether the joint of the person has LOM by using a joint point of the person estimated in the person calculation part; and
   a compensatory movement determination part configured to determine whether the joint of the person has compensatory movement by using the joint point of the person estimated in the person calculation part.

In addition, in the method for evaluating and analyzing musculoskeletal injuries through 3D dynamic joint range of motion testing, and postural abnormalities through static posture testing according to an aspect of the present disclosure, the process (a) may include selecting at least one of posture testing or motion injury testing.

In addition, in the method for evaluating and analyzing musculoskeletal injuries through 3D dynamic joint range of motion testing, and postural abnormalities through static posture testing according to an aspect of the present disclosure, the measurement device may be provided with a measurement mechanism which is capable of measuring a point where the person is positioned and which is mounted such that a minimum of one to a maximum of eight measurement mechanisms are provided, and
the measurement mechanism may be configured by selecting at least one of a camera, a sensor, an X-ray device, and a radiation device.

In addition, in the method for evaluating and analyzing musculoskeletal injuries through 3D dynamic joint range of motion testing, and postural abnormalities through static posture testing according to an aspect of the present disclosure, the server may include a database in which measurement information of the person, testing result information, and algorithm information are prestored and which is configured to construct big data on the basis of the prestored data.

In addition, in the method for evaluating and analyzing musculoskeletal injuries through 3D dynamic joint range of motion testing, and postural abnormalities through static posture testing according to an aspect of the present disclosure, the process (c) may include:
(c1) estimating a main joint of the person's body through the person calculation part and reconstructing the main joint of the person's body by three-dimensionalizing (the X, the Y, and the Z axes) the main joint of the person's body;
(c2) detecting a postural abnormality through the static posture of a plurality of joints or detecting whether there is a motion injury for each muscle that is calculated; and
(c3) calculating, by the testing result calculation part, the testing result of the person on the basis of information detected in the data analysis part.

In addition, in the method for evaluating and analyzing musculoskeletal injuries through 3D dynamic joint range of motion testing, and postural abnormalities through static posture testing according to an aspect of the present disclosure, the motion injury detection part may be configured to determine whether the person has LOM or compensatory movement by using a disease syndrome algorithm prestored in the database.

In addition, in the method for evaluating and analyzing musculoskeletal injuries through 3D dynamic joint range of motion testing, and postural abnormalities through static posture testing according to an aspect of the present disclosure, the postural abnormality detection part may be configured to determine whether the person has a postural abnormality by using a posture imbalance algorithm prestored in the database.

In addition, in the method for evaluating and analyzing musculoskeletal injuries through 3D dynamic joint range of motion testing, and postural abnormalities through static posture testing according to an aspect of the present disclosure, the process (d) may include
outputting a comprehensive and detailed testing result from the testing result calculation part on the basis of information calculated through the process (c), and
the comprehensive result may include a testing selection type, a three-dimensional posture (joint reconstruction), a posture type, an improvement degree in posture, an exercise type, a mitigation degree in motion injury, an average score, and a comprehensive score.

In addition, in the method for evaluating and analyzing musculoskeletal injuries through 3D dynamic joint range of motion testing, and postural abnormalities through static posture testing according to an aspect of the present disclosure, the motion injury detection part may determine that the person is normal when the LOM determination part and the compensatory movement determination part determine that there is no LOM and there is no compensatory movement, and
the motion injury detection part may determine that there is the syndrome for a corresponding joint when the LOM determination part determines that there is LOM or when the compensatory movement determination part determines that there is compensatory movement.

In addition, in the method for evaluating and analyzing musculoskeletal injuries through 3D dynamic joint range of motion testing, and postural abnormalities through static posture testing according to an aspect of the present disclosure, in the motion injury detection part, when the LOM determination part determines that the joint does not have LOM but the compensatory movement determination part determines that there is compensatory movement,
a final degree of compensatory movement may be calculated by comparing a degree of compensatory movement determined in the compensatory movement determination part with data prestored in the database, and a symptom of a disease and a severity of the symptom (whether the symptom is severe, moderate, or mild) may be determined according to a calculated degree value of compensatory movement.

These solutions will be more clearly understood from the following detailed description of the present disclosure when taken in conjunction with the accompanying drawings.

The terms and words used in the present specification and claims should not be interpreted as being limited to typical meanings and dictionary definitions, but should be interpreted as having meanings and concepts relevant to the technical scope of the present disclosure based on the rule according to which an inventor can appropriately define the concept of the term to describe most appropriately the best method he or she knows for carrying out the present disclosure.

### Advantageous Effects

According to an aspect of the present disclosure, the accuracy and the reliability of the posture testing or the motion injury testing about the body of the person may be improved.

In addition, according to an aspect of the present disclosure, since the rehabilitation exercise and the preventive exercise suitable for each individual are recommended according to whether there are the postural abnormality, the LOM, and the compensatory movement and guide videos for the rehabilitation exercise and the preventive exercise are provided together, there is an effect that the participation rate and the efficiency of the rehabilitation exercise and the preventive exercise may be increased.

### Description of Drawings

FIG. 1 is a schematic view illustrating a relationship between a measurement device and a server of a posture or a motion injury testing system according to an embodiment of the present disclosure.
FIG. 2 is a block diagram illustrating components of the server of the posture or the motion injury testing system according to an embodiment of the present disclosure.
FIG. 3 is a block diagram illustrating components of a person calculation part of the posture or the motion injury testing system according to an embodiment of the present disclosure.

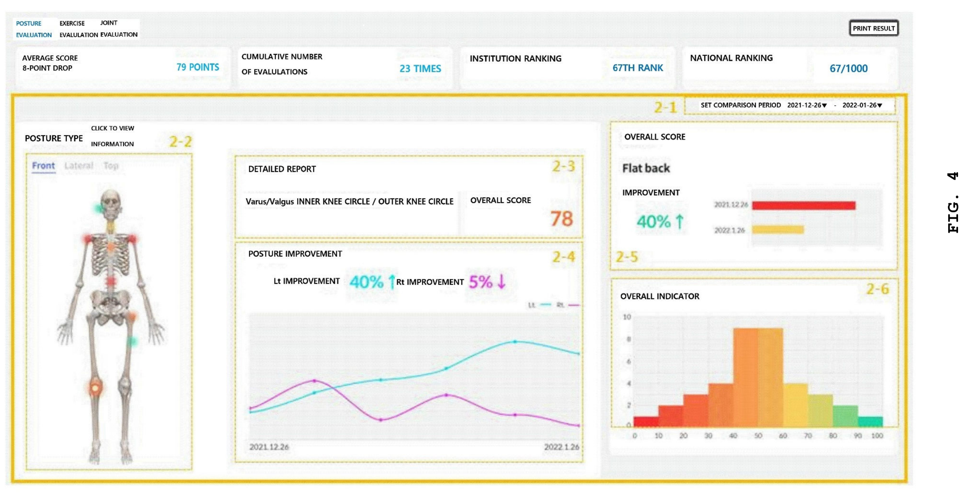

FIG. 4 to FIG. 6 are exemplary views illustrating comprehensive testing results in a data analysis part of the posture or the motion injury testing system and method according to an embodiment of the present disclosure.
FIG. 7 is a flowchart illustrating a method for evaluating and analyzing musculoskeletal injuries through 3D dynamic joint range of motion testing, and postural abnormalities through static posture testing according to an embodiment of the present disclosure.
FIG. 8 is a flowchart illustrating a method of calculating a person in the method for evaluating and analyzing musculoskeletal injuries through 3D dynamic joint range of motion testing, and postural abnormalities through static posture testing according to an embodiment of the present disclosure.

### Mode for Invention

The specific viewpoints and specific technical features of the present disclosure will become apparent from the following specific description and an embodiment related to the accompanying drawings. In the present specification, in relation to adding reference numerals to elements of each drawing, the same elements have the same reference numerals although they are indicated on different drawings. In addition, in describing an embodiment of the present disclosure, when it is determined that a detailed description of a related known configuration or function may make the subject matter of the present disclosure unclear, a detailed description thereof will be omitted.

In addition, in describing the components of the present disclosure, terms such as first, second, A, B, (a), and (b) may be used. These terms are only used to distinguish one component from another component, and the nature, sequence, or order of the corresponding component is not limited by the terms. When a component is described as being "connected", "coupled", or "linked" to another component, the component may be directly connected or linked to the other component, or other components may be "connected", "coupled", or "linked" to therebetween.

Hereinafter, an embodiment of the present disclosure will be described in detail with reference to the accompanying drawings.

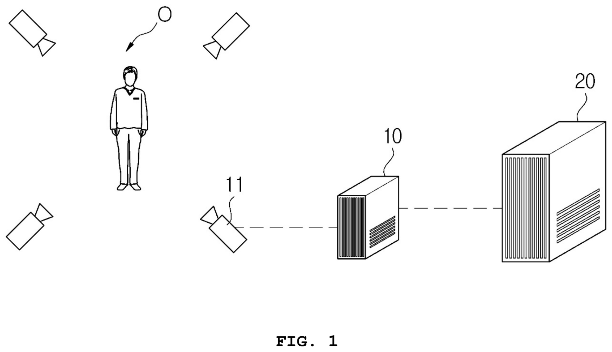

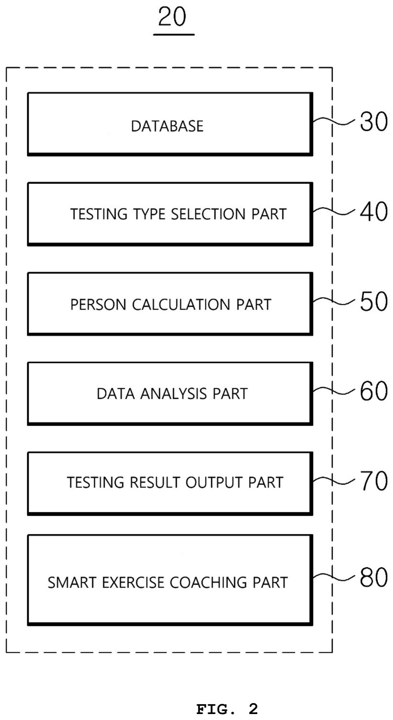

As illustrated in FIG. 1 and FIG. 2, a posture or a motion injury testing system according to an embodiment of the present disclosure may include a measurement device 10 configured to measure body information of an person O from multiple viewpoints and to collect information about the person O in three dimensions, and may include a server 20 configured to determine whether the person O has a postural abnormality or a motion injury on the basis of the information measured in the measurement device 10.

In the measurement device 10, a minimum of one to a maximum of eight measuring mechanisms 11 capable of measuring all directions corresponding to 360 degrees with respect to a point where the person O is positioned may be mounted. In the present disclosure, five measurement mechanisms 11 may be mounted at point corresponding to a front side, a rear side, a left side, and a right side with respect to the person, but may be applied in various manners according to a photographing environment or a situation.

Such a measurement mechanism 11 may be configured by selecting any one of a camera, a sensor, an X-ray device, and a radiation device, and may include the camera and the sensor in the present disclosure.

The server 20 may select at least one of posture testing or motion injury testing (otherwise, muscle injury testing or sports injury testing) for the person on the basis of the information provided by the measurement device 10, and may calculate whether the person has a postural abnormality or a motion injury.

As illustrated in FIG. 2, such a server 20 may include a database 30, a testing type selection part 40, a person calculation part 50, a data analysis part 60, a testing result output part 70, and a smart exercise coaching part 80.

A basic table required for posture testing or motion injury testing, measurement information of a previously measured person, testing result information, and algorithm information are prestored in the database 30. Furthermore, the database 30 is configured to construct big data on the basis of the prestored information, and may be used to calculate testing type information of the person in the person calculation part 50.

The testing type selection part 40 may provide at least one choice so that at least one of testing types of the person is capable of being selected. In the present disclosure, the testing type selection part 40 may be configured such that at least one of posture testing or motion injury testing is capable of being selected.

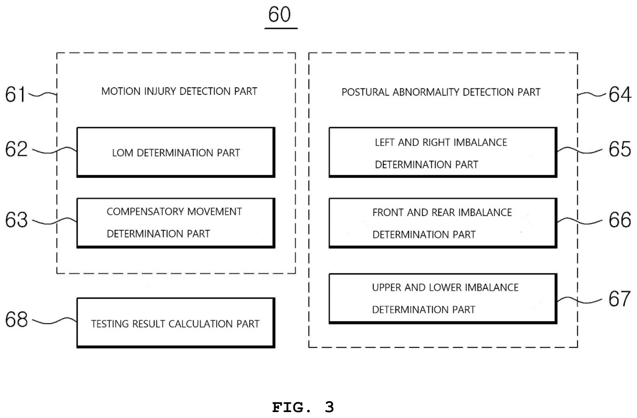

As illustrated in FIG. 3, the person calculation part 50 may perform a function of calculating person information including information about joints, muscles, and postures of the person on the basis of information received from the measurement device 10 and on the basis of the testing type selected in the testing type selection part 40.

In addition, the person calculation part 50 may perform the testing selected in the testing type selection part 40, may calculate three-dimensional (X, Y, and Z axes) values of each joint of the person, and may transmit the calculated values to the data analysis part 60.

The data analysis part 60 may analyze data on the basis of the three-dimensional (the X, the Y, and the Z axes) values calculated from the person calculation part 50, and may perform a function of deriving a testing result.

Such a data analysis part 60 may include a motion injury detection part 61 and a postural abnormality detection part 64.

The motion injury detection part 61 may perform a function of determining whether there are Limitation Of Motion (LOM) and compensatory movement by testing a dynamic posture on the basis of the three-dimensional information calculated from the person calculation part 50, and may perform a function of finally determining whether there is a syndrome on the basis of the determined information. The motion injury detection part 61 may perform the functions only when the motion injury testing is selected in the testing type selection part 40.

In addition, the motion injury detection part 61 may detect whether there is an imbalance in the LOM and the compensatory movement estimated among a disease syndrome algorithm stored in the database 30, the disease syndrome algorithm may be constructed by previously inputting a flowchart related to a syndrome according to a position of a joint of a person in the database 30, and the disease syndrome algorithm is a self-manufactured algorithm.

Such a motion injury detection part 61 may further include a LOM determination part 62 configured to determine whether the person has LOM at a joint by using a joint point of the person estimated in the person calculation part 50, and may further include a compensatory movement determination part 63 configured to determine whether the person has compensatory movement at the joint point of the person estimated in the person calculation part 50.

The LOM determination part 62 and the compensatory movement determination part 63 determine whether a person has or does not have LOM at a joint and whether the person has or does not have compensatory movement. More specifically, for example, 1) when the LOM determination part 62 and the compensatory movement determination part 63 determine that a joint of an person has LOM and compensatory movement, it can be determined that the joint of a corresponding portion has a syndrome, and 2) when the LOM determination part 62 and the compensatory movement determination part 63 determine that a joint of an person has LOM but does not have compensatory movement, it can be determined that the joint of a corresponding portion has a syndrome. In addition, 3) when the LOM determination part 62 and the compensatory movement determination part 63 determine that a joint of an person does not have LOM but has compensatory movement, it can be determined that there is a syndrome according to a calculation value of the compensatory movement, and 4) when the LOM determination part 62 and the compensatory movement determination part 63 determine that a joint of an person does not have LOM and compensatory movement, it can be determined that there is no syndrome in the joint of a corresponding portion.

Here, 3) when it is determined that a joint does not have LOM but has compensatory movement, a symptom of the syndrome and the severity of the symptom (whether the symptom is severe, moderate, or mild) may be determined according to the degree of the compensatory movement.

That is, the motion injury detection part 61 may determine whether there is a syndrome on the basis of the presence or absence of LOM and the presence or absence of compensatory movement that are determined by the LOM determination part 62 and the compensatory movement determination part 63.

The postural abnormality detection part 64 performs a function of detecting whether there is an imbalance in a static posture of a plurality of joints and in each muscle, and the function may be performed only when the postural abnormality testing is selected in the testing type selection part 40.

In addition, the postural abnormality detection part 64 may detect whether there is an imbalance in an estimated posture among a posture imbalance algorithm prestored in the database 30, the posture imbalance algorithm may be constructed by previously inputting a flowchart related to a syndrome according to a position of a joint of a person in the database 30, and the posture imbalance algorithm is a self-manufactured algorithm.

Such a postural abnormality detection part 64 may include a left and right imbalance determination part 65, a front and rear imbalance determination part 66, and an upper and lower imbalance determination part 67.

The left and right imbalance determination part 65 may determine whether there is a left and right imbalance in a posture of a person, the front and rear imbalance determination part 66 may determine whether there is a front and rear imbalance in a posture of a person, and the upper and lower imbalance determination part 67 may determine whether there is an upper and lower imbalance in a posture of a person.

That is, the postural abnormality detection part 64 may determine whether there is a postural imbalance in left, right, front, rear, upper, and lower sides of a person.

In addition, results analyzed in the motion injury detection part 61 and the postural abnormality detection part 64 may be calculated as numerical values in a testing result calculation part 68, and the numerical values calculated in the testing result calculation part 68 may be transmitted to the testing result output part 70.

The testing result output part 70 may perform a function of outputting the result values (the numerical values calculated in the testing result calculation part) calculated in the motion injury detection part 61 and the postural abnormality detection part 64 to a GUI and, separately, may output the result values as a comprehensive testing result and may provide the comprehensive testing result to the person.

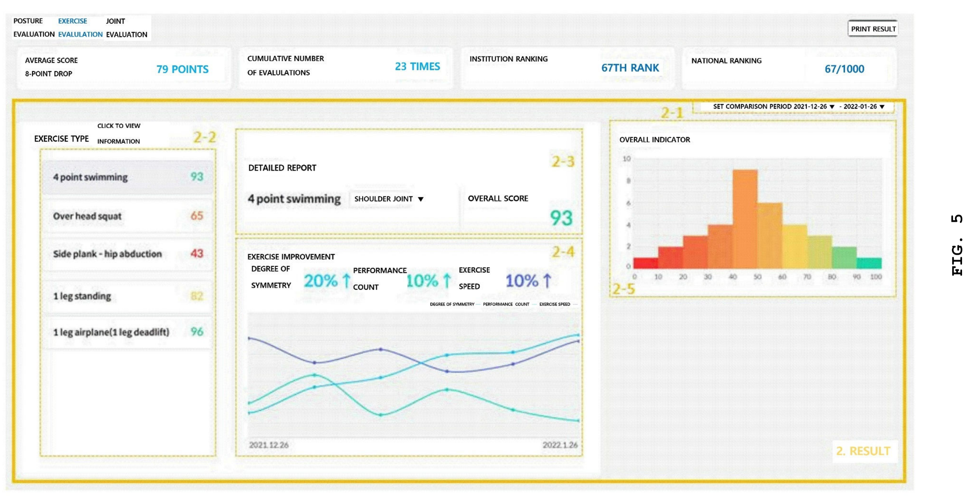

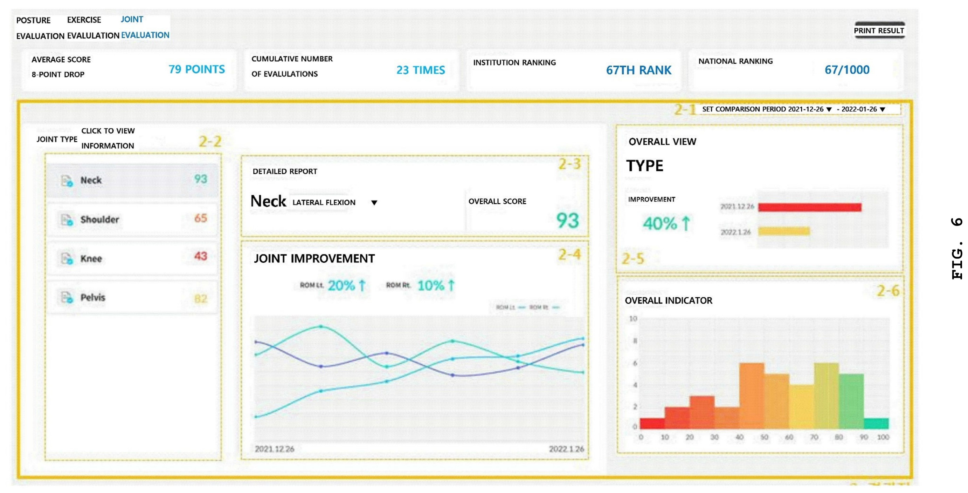

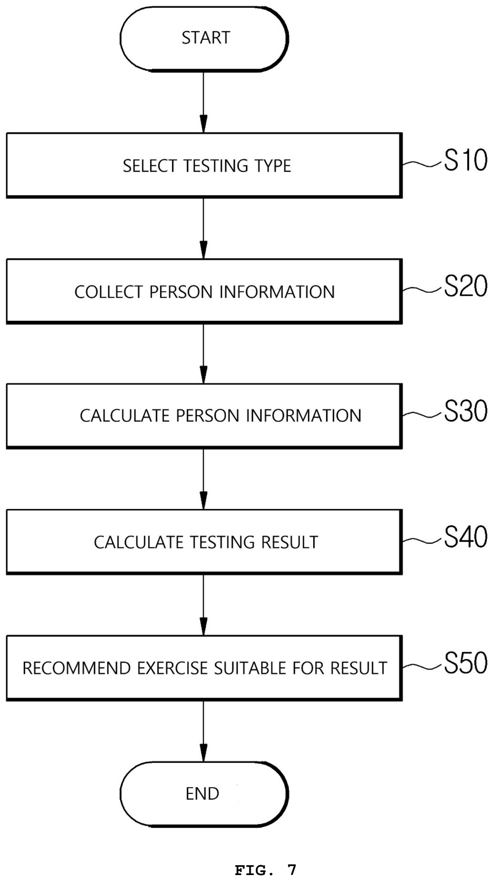

Here, as illustrated in FIG. 5 to FIG. 7, the comprehensive testing result may include a testing selection type, a three-dimensional posture (joint reconstruction), a posture type, an improvement degree in posture, an exercise type, a mitigation degree in motion injury, an average score, and a comprehensive score.

The smart exercise coaching part 80 may search rehabilitation exercise information or preventive exercise information to be recommended to the person from the database 30 on the basis of information calculated in the data analysis part 60, and may additionally output the rehabilitation exercise information or the preventive exercise information when the comprehensive testing result is output.

Here, when the motion injury detection part 61 and the postural abnormality detection part 64 determine that a person has a syndrome or a postural abnormality, the smart exercise coaching part 80 may perform a function of recommending a rehabilitation exercise or a preventive exercise suitable for the determined syndrome.

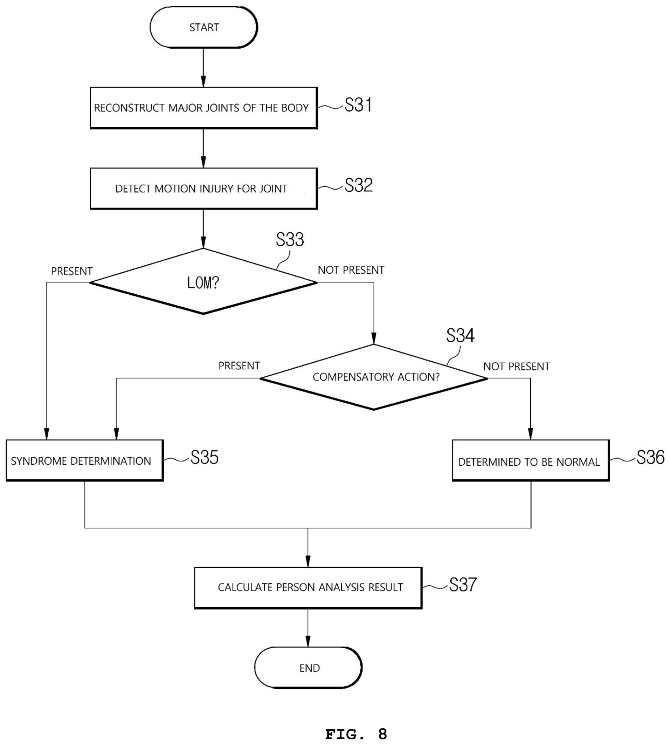

As illustrated in FIG. 8, a method for evaluating and analyzing musculoskeletal injuries through 3D dynamic joint range of motion testing, and postural abnormalities through static posture testing, which uses the posture or the motion injury testing system, may include: selecting, through the testing type selection part 40, a testing type for which an person intends to be tested (S10); collecting, from the person, information suitable for the selected testing type (S20); calculating person information including information about a joint, a muscle, and a posture of the person on the basis of big data constructed in the database 30 and on the basis of testing type information selected in the testing type selection part 40, and determining whether there is LOM or compensatory movement about the joint, the muscle, and the posture of the person in the data analysis part 60 on the basis of calculated information, thereby determining whether there is a syndrome (S30); calculating a testing result in the testing result output part 70 on the basis of information analyzed in the data analysis part 60 (S40); and recommending information about a rehabilitation exercise or a preventive exercise on the basis of information calculated in the testing result output part 70 (S50).

First, before testing is performed, the person may select testing for which the person intends to be tested, may select at least one of the posture testing or the motion injury testing and testing about a posture or a motion injury of the person may be performed. In the present disclosure, it is described that the motion injury testing is selected and performed (S10).

After the testing type is selected, 360 degrees of all directions of the person may be photographed through the measurement mechanisms at the front side, the rear side, the left side, and the right side of the person through the measurement device 10, and the measured information may be transmitted to the server 20. Here, the measurement device 10 and the server 20 may be connected to a wired or wireless network (S20).

The information measured in the measurement device 10 may be used for calculating the person information through the person calculation part 50, and whether the person has a syndrome (or a disease) may be determined on the basis of the calculated information (S30).

Specifically, as illustrated in FIG. 9, the main joints of the human body may be estimated in the person calculation part 50, the main joints of the human body may be reconstructed on the basis of the estimated information (S31), and whether the joint has a motion injury may be detected by using the disease syndrome algorithm (S32).

When it is determined that the joint of the person does not have LOM and does not have compensatory movement, it can be determined that the person being tested is normal (S36). However, when it is determined that the joint of the person has at least one of LOM or compensatory movement, it can be determined that a corresponding joint has a syndrome (S35).

For example, when it is determined that the joint does not have LOM but has compensatory movement, it can be determined that the person does not have a syndrome. However, a direction in which the person moves may be additionally calculated, and a symptom and the severity of the symptom (whether the symptom is severe, moderate, or mild) may be determined.

After whether the person has the syndrome or whether the person is normal is determined (S35, S36), the testing result may be numerically calculated through the testing result calculation part 68, and the comprehensive testing result may be calculated in the testing result output part 70 on the basis of the calculated numerical value (S37).

The testing result output part 70 may additionally calculate the comprehensive test result on the basis of information of whether the person has the syndrome or whether the person is normal, and the comprehensive testing result may include at least one of the testing selection type, the three-dimensional posture (joint reconstruction), the posture type, the improvement degree in posture, the exercise type, the mitigation degree in motion injury, the average score, and the comprehensive score (S40).

Subsequently, the smart exercise coaching part 80 may recommend the rehabilitation exercise or the preventive exercise on the basis of the testing result calculated in the testing result output part 70. More specifically, when it is determined that the joint has LOM (S33), it is determined that the person has the disease, and the smart exercise coaching part 80 may recommend the rehabilitation exercise suitable for the determined disease by using information prestored in the database. Furthermore, when it is determined that the joint does not have LOM (S34), it is determined that the person does not have the disease, but the symptom and the severity of the symptom may be determined by additionally calculating the direction in which the person moves, and the smart exercise coaching part 80 may recommend the preventive exercise on the basis of information prestored in the database.

That is, according to an embodiment of the present disclosure, the accuracy and the reliability of the posture testing or the motion injury testing about the body of the person may be improved.

In addition, according to an embodiment of the present disclosure, since the rehabilitation exercise and the preventive exercise suitable for each individual are recommended according to whether there are the postural abnormality, the LOM, and the compensatory movement and guide videos for the rehabilitation exercise and the preventive exercise are provided together, there is an effect that the participation rate and the efficiency of the rehabilitation exercise and the preventive exercise may be increased.

Although the present disclosure has been described in detail through an embodiment, this is intended to describe the present disclosure in detail, and the method for evaluating and analyzing musculoskeletal injuries through 3D dynamic joint range of motion testing, and postural abnormalities through static posture testing according to the present disclosure is not limited to the embodiment. The terms such as "includes", "forms", or "comprises" described above mean that a corresponding component may be included unless stated otherwise, and, accordingly, other components may be further included, not excluded. All terms, including technical or scientific terms, unless defined otherwise, have the same meaning as commonly understood by one of ordinary skill in the technical field to which the present disclosure belongs.

In addition, although the embodiment of the present disclosure has been disclosed for illustrative purposes, those skilled in the art will appreciate that various modifications, additions and substitutions are possible, without departing from the scope and spirit of the present disclosure. Accordingly, embodiments and the accompanying drawings disclosed in the present disclosure are provided for describing the present disclosure and are not intended to limit the technical ideas of the present disclosure. The technical ideas of the present disclosure are not limited to the embodiments and the drawings. The scope of the present disclosure should be construed as being covered by the scope of the appended claims, and all technical ideas falling within the scope of the claims should be construed as being included in the scope of the present disclosure.

## Claims

1. A method for evaluating and analyzing musculoskeletal injuries through 3D dynamic joint range of motion testing, and postural abnormalities through static posture testing, the method comprising:
(a) selecting, by a person through a testing type selection part provided by a server, at least one testing type for which the person intends to be tested;
(b) photographing a movement and a motion of the person through a multiple viewpoints measurement device and transmitting photographed information to the server;
(c) three-dimensionalizing (X, Y, and Z axes), by an person calculation part, information comprising information of a joint, a muscle, and a posture about the person on the basis of the testing type selected in the testing type selection part and on the basis of the information collected in the multiple viewpoints measurement device, and determining, by a data analysis part, whether the person has Limitation Of Motion (LOM) or compensatory movement, thereby determining whether there is a syndrome;
(d) calculating, by a testing result calculation part, a testing result on the basis of information calculated in the data analysis part; and
(e) recommending, through a smart exercise coaching part, a rehabilitation exercise or a preventive exercise on the basis of the testing result calculated in the data analysis part,
wherein the data analysis part comprises:
a motion injury detection part configured to detect a static posture of at least one of joints of the person and to detect whether there is an imbalance for each muscle that is estimated; and
a postural abnormality detection part configured to detect whether there is an imbalance for the static posture of at least one of the joints,
wherein the motion injury detection part comprises:
a LOM determination part configured to determine whether the joint of the person has LOM by using a joint point of the person estimated in the person calculation part; and
a compensatory movement determination part configured to determine whether the joint of the person has compensatory movement by using the joint point of the person estimated in the person calculation part.

2. The method of claim 1, wherein the process (a) comprises selecting at least one of posture testing or motion injury testing.

3. The method of claim 1, wherein the measurement device is provided with a measurement mechanism which is capable of measuring a point where the person is positioned and which is mounted such that a minimum of one to a maximum of eight measurement mechanisms are provided, and the measurement mechanism is configured by selecting at least one of a camera, a sensor, an X-ray device, and a radiation device.

4. The method of claim 1, wherein the server comprises a database in which measurement information of the person, testing result information, and algorithm information are prestored and which is configured to construct big data on the basis of the prestored data.

5. The method of claim 1, wherein the process (c) comprises:
(c1) estimating a main joint of the person's body through the person calculation part and reconstructing the main joint of the person's body by three-dimensionalizing (the X, the Y, and the Z axes) the main joint of the person's body;
(c2) detecting a postural abnormality through the static posture of a plurality of joints or detecting whether there is a motion injury for each muscle that is calculated; and
(c3) calculating, by the testing result calculation part, the testing result of the person on the basis of information detected in the data analysis part.

6. The method of claim 1, wherein the motion injury detection part is configured to determine whether the person has LOM or compensatory movement by using a disease syndrome algorithm prestored in a database.

7. The method of claim 1, wherein the postural abnormality detection part is configured to determine whether the person has a postural abnormality by using a posture imbalance algorithm prestored in a database.

8. The method of claim 1, wherein the process (d) comprises outputting a comprehensive and detailed testing result from the testing result calculation part on the basis of information calculated through the process (c), and the comprehensive result comprises a testing selection type, a three-dimensional posture (joint reconstruction), a posture type, an improvement degree in posture, an exercise type, a mitigation degree in motion injury, an average score, and a comprehensive score.

9. The method of claim 1, wherein the motion injury detection part determines that the person is normal when the LOM determination part and the compensatory movement determination part determine that there is no LOM and there is no compensatory movement, and
the motion injury detection part determines that there is the syndrome for a corresponding joint when the LOM determination part determines that there is LOM or when the compensatory movement determination part determines that there is compensatory movement.

10. The method of claim 9, wherein, in the motion injury detection part, when the LOM determination part determines that the joint does not have LOM but the compensatory movement determination part determines that there is compensatory movement,
a final degree of compensatory movement is calculated by comparing a degree of compensatory movement determined in the compensatory movement determination part with data prestored in a database, and a symptom of a disease and a severity of the symptom (whether the symptom is severe, moderate, or mild) are determined according to a calculated degree value of compensatory movement.
